# EUROPEAN PATENT SPECIFICATION

(11) **EP 1 872 743 B1**
(45) Date of publication and mention of the grant of the patent: **05.08.2009**
(21) Application number: 07111254.4
(22) Date of filing: 28.06.2007
(51) Int. Cl.: A61F 2/24

(54) **Cardiovascular valve assembly with resizable docking station**
Kardiovaskuläre Ventilanordnung mit Dockstation mit veränderlicher Größe
Assemblage de valve cardiovasculaire doté d'une station d'accueil redimensionnable

(30) Priority: 29.06.2006 US 806106 P; 11.06.2007 US 760840
(43) Date of publication of application: 02.01.2008
(73) Proprietor: ValveXchange Inc., Tarzana CA 91356-4312 (US)
(72) Inventor: Vesely, Ivan, Larkspur, CO 80118 (US)
(74) Representative: Reitstötter - Kinzebach

(56) References cited:
- WO-A-20/06127756
- US-A1- 2004 030 381
- US-B1- 6 454 799

## Description

### Field of Invention

The present invention relates generally to a cardiovascular valve assembly, and more specifically relates to a cardiovascular valve assembly comprised of a resizable base member that remains in a patient, and a valve member that is detachably mountable to the resizable base member.

### Background of the Invention

U.S. Patent Application Serial No. 11/296,899 ("Cardiovascular Valve Assembly"), filed December 8, 2005 (published as U.S. Patent Application Publication US 2006/0136052 on June 22, 2006), discloses an exchangeable cardiovascular valve assembly comprised of an exchangeable valve member, which includes leaflet components, and a base member (also referred to as a "docking station"). The exchangeable valve member is detachably mounted to the base member. The base member is intended for permanent implantation within a patient. Accordingly, the base member remains inside the patient during subsequent exchanges of valve members.

A valve member may require periodic replacement due to wear. Accordingly, the current valve member is detached from the permanent base member and a new valve member is mounted thereto. It may also be necessary to replace the current valve member because the patient has grown and requires a valve member having larger dimensions. In this regard, children need frequent valve member replacements as they grow to full adult size. However, existing base members have a fixed diameter, thus preventing a simple exchange of valve members with different dimensions.

One purpose of the present invention is to address the needs of a child patient who may have valve problems (e.g., pulmonary stenosis). Currently, these child patients are treated surgically or with a new generation of catheter deployable valves. However, catheter deployable valves are permanent devices. There is no provision for replacing such valves percutaneously when the valve fails or needs to be replaced due to sizing issues. It is generally thought that a single percutaneous deployment of a valve will avoid the first surgery and allow the patient to mature and recover for a few years until it is time for the first revision surgery.

The present invention address the drawbacks of existing cardiovascular valve assemblies by providing a valve assembly including a resizable base member that is adaptable to receive valve members of various sizes.

WO 2006/127756 A2, which forms prior art pursuant to Art. 54(3) and (4) EPC, describes a component-based valve prosthesis that can be implanted during a surgical procedure. The valve comprises a support structure and a valve member that may be connected to the support structure. The support structure may take the form of a balloon-expandable stent. The support structure is provided with a coupling means for attachment to the valve member, thereby fixing the position of the valve member in the body.

Furthermore, US 2004/0030381 A1 describes a heart valve prosthesis having a frame that includes one outwardly extending annular flange adjacent to a suturing ring or a band comprising an inwardly extending annular flange that is capable of engagement with the annular flange of the frame.

Moreover, US 6,454,799 B1 describes an expandable heart valve that includes an annular tissue-engaging base and a subassembly having an elastic wireform and a plurality of leaflets connected thereto. The annular base and subassembly are separately stored and connected just prior to delivery to the host annulus.

### Summary of the Invention

In accordance with one aspect of the present invention, there is provided a valve assembly comprising: a base member deformable to increase the dimensions thereof; and a valve member including a valve frame and a plurality of leaflets supported by the valve frame, said valve member detachably mountable to said base member.

In accordance with another aspect of the present invention, there is provided a valve assembly comprising: (a) a base member deformable to increase the dimensions thereof, said base member including: a tubular body having an adjustable diameter, a plurality of mounting elements attached to the tubular body, a plurality of securing elements attached to the tubular body; and (b) a valve member detachably mountable to said base member, said valve member including: a valve frame and a plurality of leaflets supported by the valve frame, a plurality of coupling elements attached to the valve frame, each said coupling element engageable with a respective mounting element to detachably mount said valve member to said base member, and a plurality of fingers attached to the valve frame, each finger capturable by a respective securing element of the base member.

An advantage of the present invention is the provision of a cardiovascular valve assembly including a base member that is expandable to receive valve members of varying dimensions.

Still another advantage of the present invention is the provision of a cardiovascular valve assembly including an exchangeable valve member that is mountable to an expandable base member.

These and other advantages will become apparent from the following description of embodiments of the present invention taken together with the accompanying drawings and the appended claims.

### Brief Description of the Drawing

The invention may take physical form in certain parts and arrangement of parts, embodiments of which will be described in detail in the specification and illustrated in the accompanying drawing which form a part hereof, and wherein:

FIG. 1 illustrates a fully assembled valve assembly according to an embodiment of the present invention;

FIG. 2 is an exploded side elevational view of a valve assembly according to the present invention, including an unexpanded base member and a valve member of a first dimension detached therefrom.

FIG. 3 is an exploded said elevational view of a valve assembly according to the present invention, including an expanded base member and a valve member of a second dimension detached therefrom;

FIG. 4 is a perspective view of a valve member according to the present invention, with leaflet components removed therefrom;

FIG. 5 is an enlarged perspective view of engagement means for attaching a valve member to a base member;

FIG. 6 shows the inner surface of an unexpanded base member in a planar profile;

FIG. 7 shows the inner surface of an expanded base member in a planar profile;

FIG. 8 is an enlarged side view of a securing element of the base member, according to a first embodiment of the securing element;

FIG. 9 is a perspective view of the securing element of FIG. 8;

FIG. 10 is an enlarged side view of a securing element of the base member, according to a second embodiment of the securing element;

FIG. 11 is a perspective view of the securing element of FIG. 10;

FIG. 12 is an enlarged side view of a securing element of the base member, according to a third embodiment of the securing element;

FIG. 13 is a perspective view of the securing element of FIG. 12; and

FIG. 14 is an enlarged perspective view of engagement means for attaching a valve member to a base member, according to an alternative embodiment of the engagement means.

### Detailed Description of the Invention

The present invention provides improvements to valve devices such as those disclosed in U.S. Patent Application Serial No. 11/296,899, filed December 8, 2005.

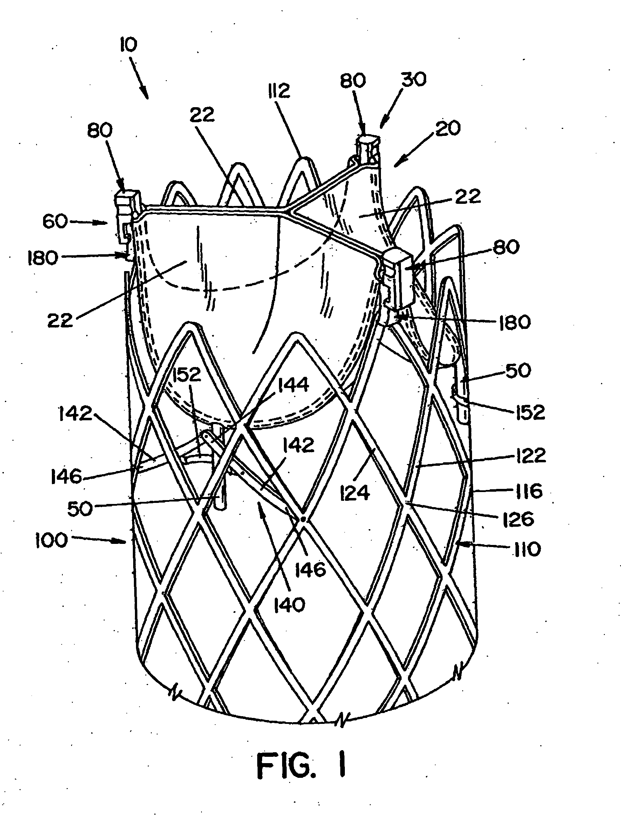

Referring now to the drawings wherein the showings are for the purpose of illustrating embodiments of the present invention only and not for the purposes of limiting same, FIG. 1 illustrates a fully assembled cardiovascular valve assembly 10 according to a first embodiment of the present invention. Valve assembly 10 is basically comprised of a replaceable valve member 20 and an expandable base member 100.

Valve member 20 is generally comprised of a valve frame 30 and a plurality of pericardial leaflets 22 supported by valve frame 30. Base member 100 is permanently installed in a patient by conventionally known means. Valve member 20 is detachably mountable to base member 100, as will be described in detail below. It should be understood that valve member 20 and base member 100 may take forms other than as illustrated herein.

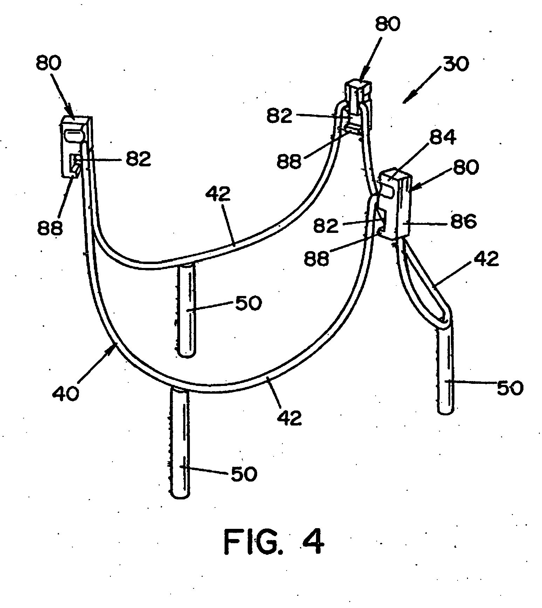

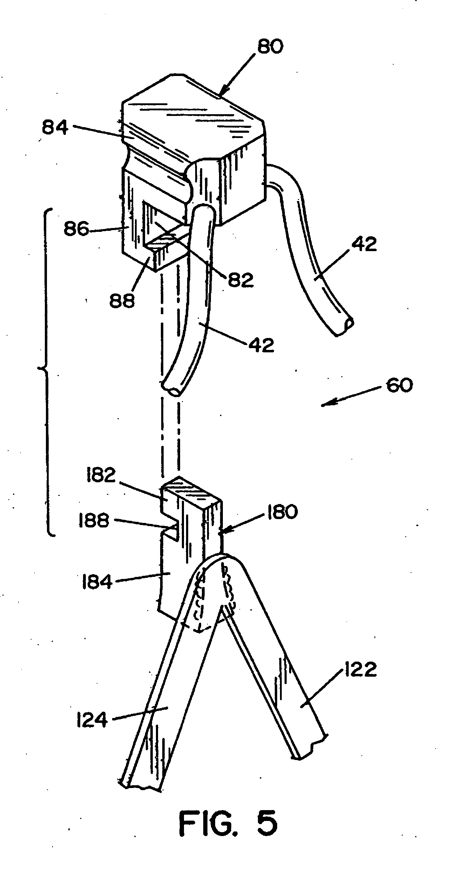

Referring now to FIG. 4, there is shown valve frame 30 with leaflets 22 removed therefrom for improved clarity. Valve frame 30 includes a wireform 40, comprised of a plurality of wireform sections 42, and coupling elements 80. Each wireform section 42 has a generally arcuate shape, and extends between coupling elements 80. As best seen in FIG. 5, coupling elements 80 are comprised of an upper section 84, a downward extending section 86 and an inward extending tab 88. An inward facing recess 82 is defined by upper section 84, downward extending section 86 and tab 88. Fingers 50 extend downward from each wireform section 42. In the illustrated embodiment, each finger 50 is located approximately in the center of wireform section 42 between adjacent coupling elements 80.

Wireform 40 may be formed of a single continuous material that comprises wireform sections 42, or may be formed of individual discrete wireform sections 42 that are joined together at coupling elements 80. Wireform 40 is preferably made of a medical grade metal wire with suitable elasticity, such as Algiloy, nitinol, stainless steel, platinum, gold, titanium, other biocompatible metals, and combinations thereof. It should be understood that a preferred material for wireform 40 has an elasticity such that the material returns to its original shape after being deformed. However, it is contemplated that a material that does not return to its original shape after deformation could also be suitably used.

Base member 100 is generally comprised of a tubular body 110 and a mounting element 180. Tubular body 110 has a first end 112 and a second end (not shown), and a wall 116 disposed between first end 112 and the second end. First end 112 is the outflow end, while the second end is the inflow end, or vice versa. Wall 116 is formed by a plurality of intersecting elongate members 122 and 124. At least some of the elongate members 122, 124 intersect with one another at intersection points 126.

Body 110 has a first diameter and is deformable to expand to a second diameter larger than the first diameter. Body 110 is expanded by application of a radially, outwardly extending force from the interior of body 110. For example, body 110 may be expanded to the second diameter by inflating a balloon (e.g., a balloon catheter) located within the interior of body 110. The second diameter of body 110 is variable and dependent upon the amount of force applied to the body 110.

The plurality of elongate members 122, 124 may be a plurality of wires. The wires may be fixedly secured to one another where the wires intersect with one another to form a wire mesh. It is contemplated that the plurality of elongate members 122, 124 may take forms other than as illustrated.

Elongate members 122, 124 are made of a material having the required strength and elastic characteristics to allow expansion of body 110 (e.g., allow plastic deformation) and to allow body 110 to retain an expanded configuration. By way of example, and not limitation, suitable materials for the fabrication of body 110 include silver, tantalum, stainless steel, gold, and titanium.

It is contemplated that in one embodiment of the present invention, tubular body may include mechanical tabs, teeth and/or hooks to prevent body 110 from returning to a smaller diameter after expansion, such as that used in a ratchet or in some intravascular stents.

The plurality of elongate members 122, 124 are fixedly secured to one another at intersection points 126, thereby forming a mesh tube. A wire mesh tube is formed where elongate members 122, 124 are wires. It will be appreciated that elongate members 122, 124 may be fixedly secured to one another in any conventional manner (e.g., by welding, soldering, or gluing).

As best seen in FIG. 5, mounting element 180 includes an upper section 182 and a lower section 184. Sections 182 and 184 define an outward facing notch 188. Notch 188 is dimensioned to receive tab 88 of coupling element 80. Similarly, inward facing recess 82 of coupling element 80 is dimensioned to receive upper section 182 of mounting element 180. In the illustrated embodiment, mounting elements 180 are attached to tubular body 110 at first end 112. For example, mounting elements 180 may be welded to the inner or outer surfaces of tubular body 110, or welded to the edge of first end 112.

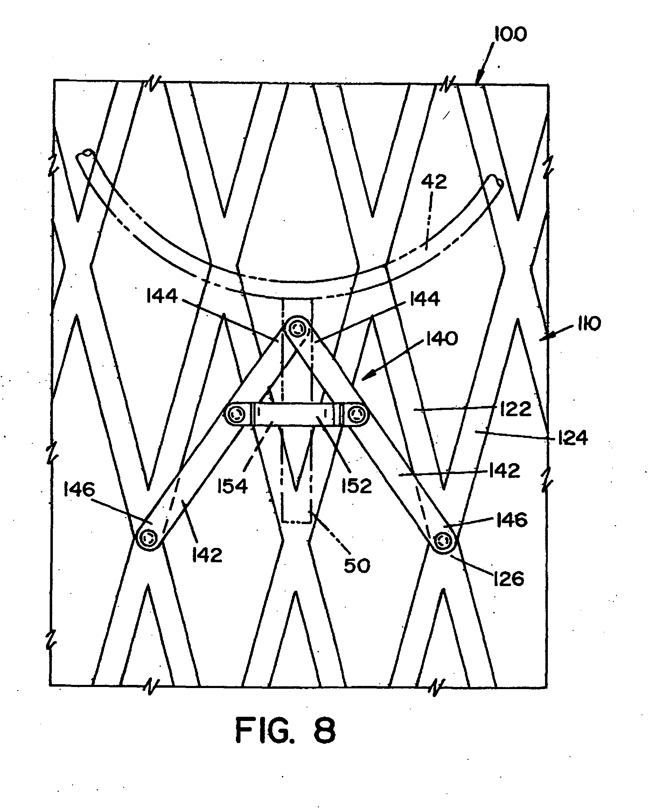

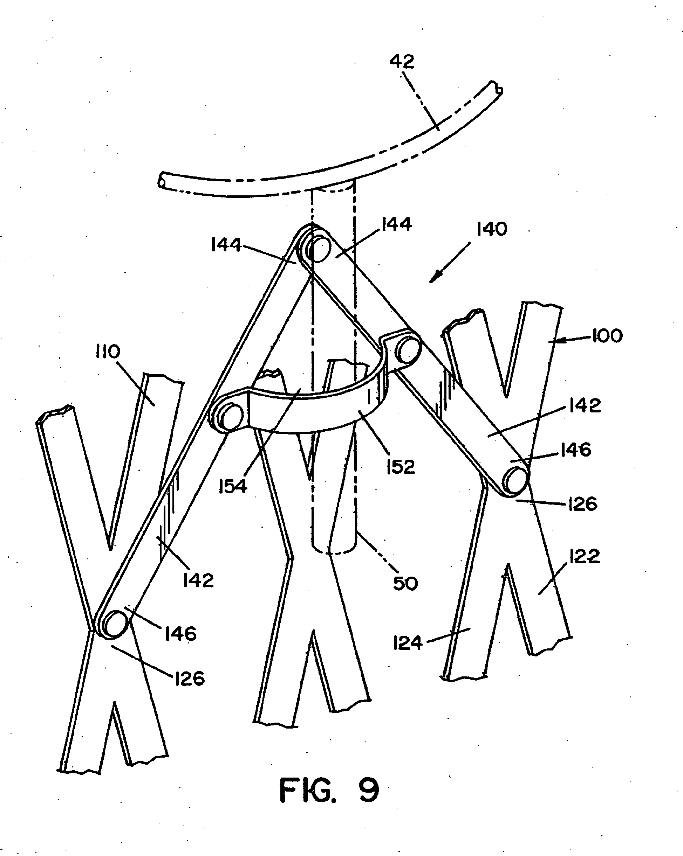

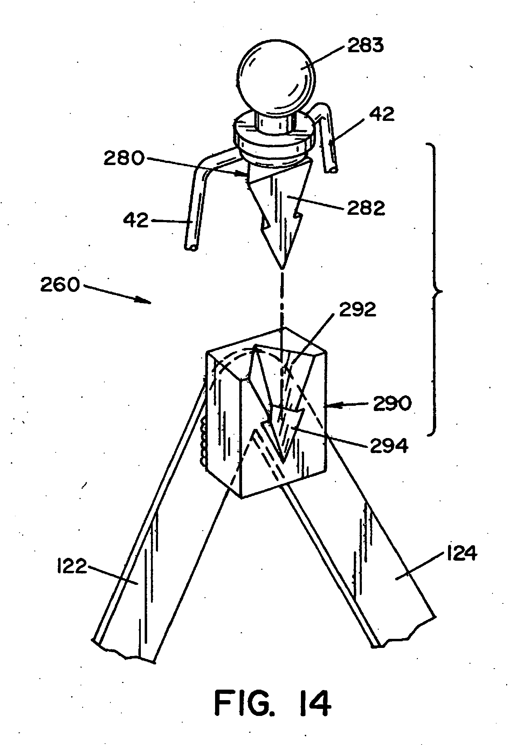

Coupling element 80 and mounting element 180 are engageable with each other, and collectively form engagement means 60 for detachably mounting valve member 20 to base member 100. FIG. 14 illustrates an engagement means 260 according to an alternative embodiment. Engagement means 260 includes a coupling member 280 having a projecting element 282 (e.g., a locking pin with a spike) and a bulb portion 283, and a mounting element 290 having a recess 292. Recess 292 is dimensioned to mate with projecting element 282 by receiving at least a portion of projecting element 282. Surface 294 of recess 292 conforms to the outward facing surface of projecting element 282. It should be appreciated that engagement means 60 may take forms other than those illustrated herein. A detailed description of engagement means 260 of FIG. 14, as well as a description of additional embodiments of suitable engagement means, is provided in U.S. Patent Application Serial No. 11/296,899, filed December 8, 2005 ("Cardiovascular Valve Assembly").Base member 100 also includes securing elements 140 for capturing fingers 50 of valve frame 30. According to the embodiment shown in FIGS. 8 and 9, each securing element 140 is comprised of a pair of arms 142, having first and second ends 144, 146, and a flexible strap 152. Arms 142 are pivotally connected to each other at first ends 144 by fastening means (e.g., a rivet or pin). Second ends 146 of arms 142 are pivotally connected by fastening means to the inner side of tubular body 110 at respective intersection points 126, as shown in FIGS. 8 and 9. Strap 152 is attached at opposite ends to arms 142 by fastening means. Strap 152 extends between the pair of arms 142, thereby forming an opening 154. Opening 154 is dimensioned to receive a finger 50 of valve frame 30.

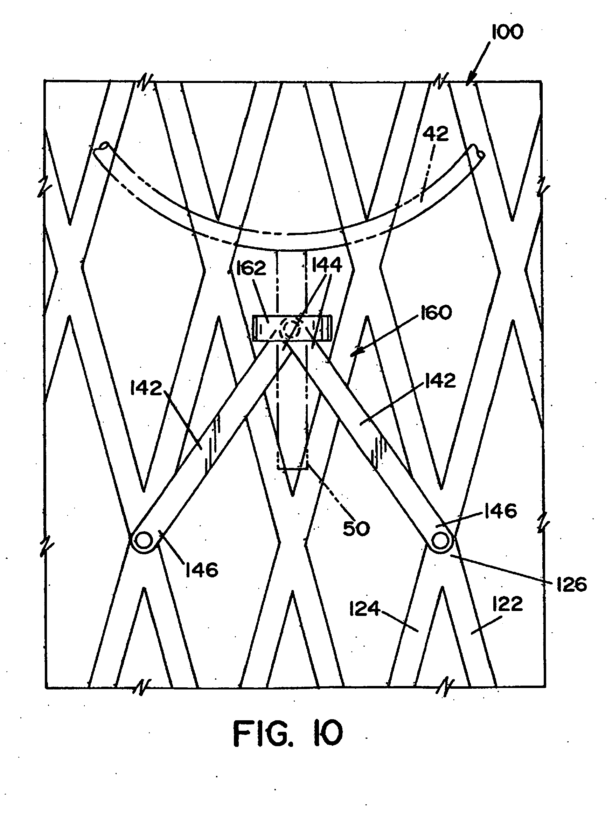

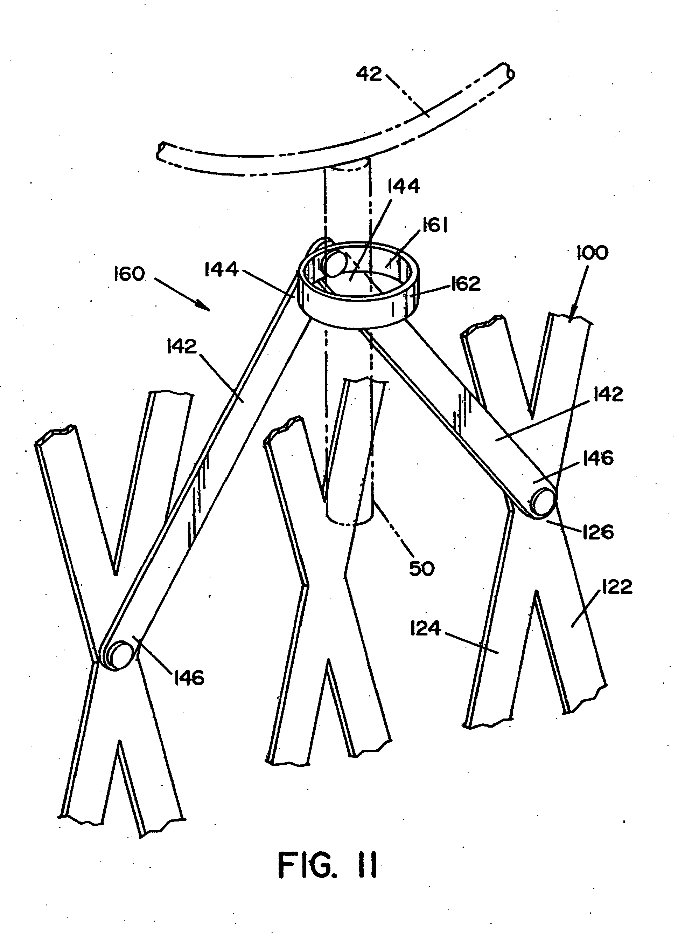

FIGS. 10 and 11 illustrate a securing element 160 according to an alternative embodiment. Securing element 160 is comprised of a pair of arms 142, as discussed above, and an annular band 162. Band 162 is attached to arms 142 by the same fastening means that connects arms 142 together at first ends 144. Band 162 defines an opening 164 dimensioned to receive a finger 50 of valve frame 30.

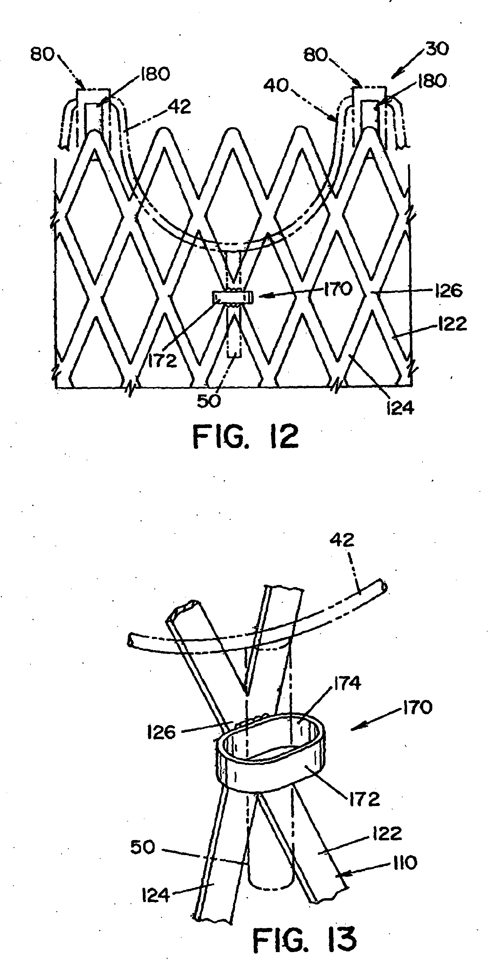

FIGS. 12 and 13 illustrate a securing element 170 according to yet another alternative embodiment. Securing element 170 is comprised of an annular band 172 fastened directly to the inner side of tubular body 110. As illustrated, band 172 is welded directly to an elongate member 122 or 124, preferably at an intersection point 126. Band 172 defines an opening 174 dimensioned to receive a finger 50 of valve frame 30.

Engagement means 60, together with the securing elements, maintain valve member 20 in engagement with base member 100.

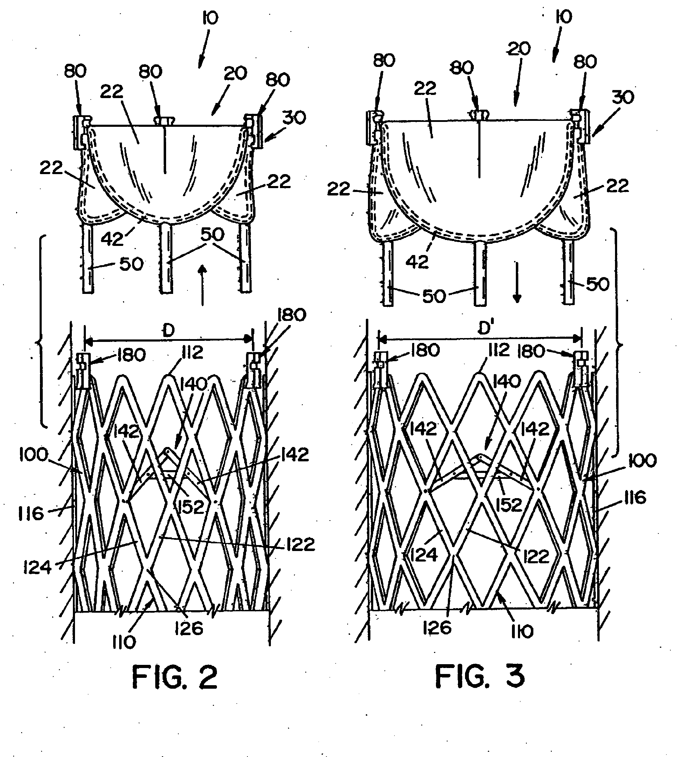

Referring now to FIG. 2, there is shown an unexpanded base member 100 having a first diameter and a detached valve member 20 having the first diameter. The diameter of valve member 20 is defined by the spacing between adjacent coupling elements 80. As indicated above, base member 100 is expandable to increase the diameter of tubular body 110, thereby dimensioning base member 100 to receive a valve member 20 having a larger diameter. FIG. 3 shows an expanded base member 100 having a second diameter and a detached valve member 20 having the second diameter, wherein the second diameter is larger than the first diameter.

The diameter of tubular body 110 may be progressively increased in stages.
In accordance with one embodiment of the present invention, body 110 has detectable limits for each stage of diameter expansion. These limits may be comprised of interlocking tabs that generate variable resistance to expansion that can be felt or measured, or markers that are visible on fluoroscopy. Alternatively, limits may be set by the maximum expanded diameter of the balloon that is used to dilate tubular body 110 to the next dilated diameter.

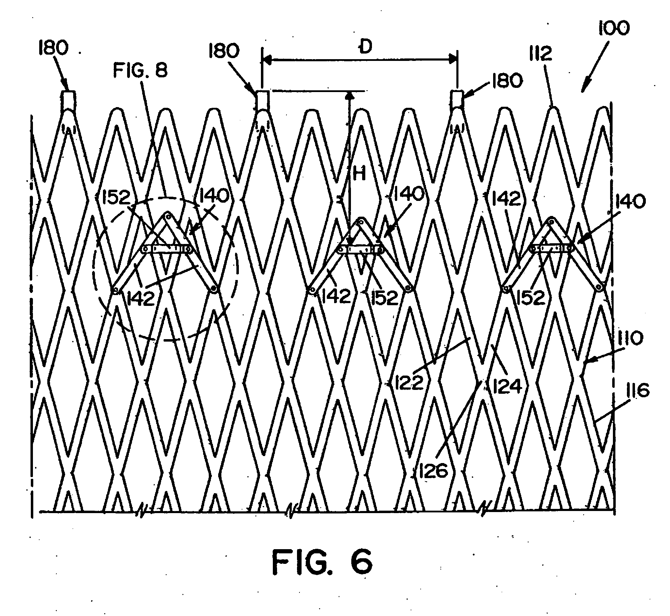

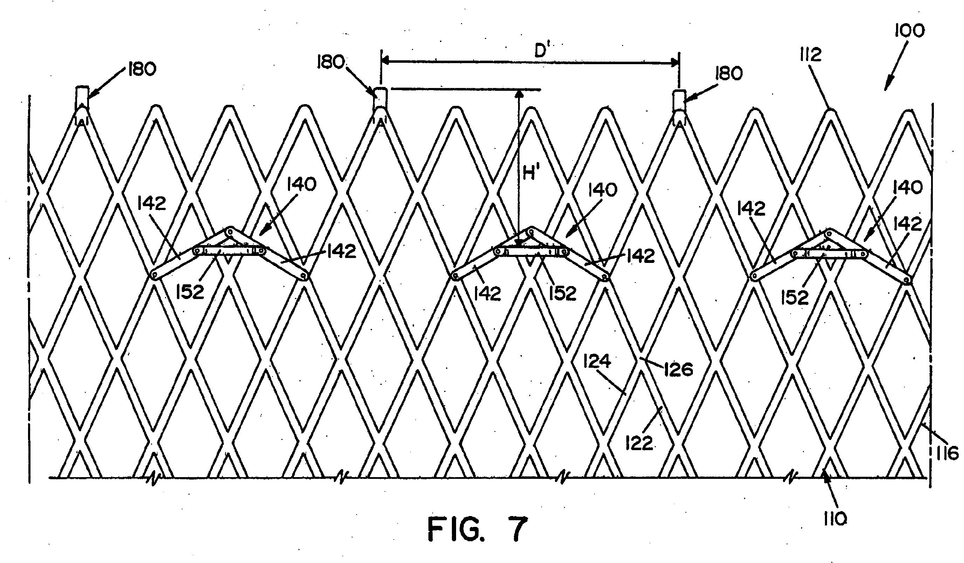

Referring to FIGS. 6 and 7, there is shown tubular body 110 in a planar provide (i.e., unrolled as a planar mesh sheet). In FIG. 6, base member 100 is unexpanded (i.e., first diameter), while in FIG. 7 base member 100 is expanded (i.e., second diameter). D and D' represent the distance between adjacent mounting elements 180. H and H' represents the distance between mounting element 180 of base member 100 and strap 152 of securing element 140.

It should be understood that upon expansion of tubular body 110 to increase the diameter thereof, the distance between the first end 112 and the second end of body 110 will decrease. In accordance with the embodiments shown in FIGS. 8, 9 and FIGS. 10, 11, the aspect ratio D'/H' is maintained to be substantially equal to the aspect ratio D/H, as base member 100 is expanded to increase the diameter of tubular body 110. As base member 100 is expanded, arms 142 of securing element 140 articulate or pivot relative to each other in a downward direction away from first end 112 of body 110, as shown in FIG. 7. As indicated above, strap 152 is formed of a flexible material that allows movement of arms 142. The movement of arms 142 substantially maintains the aspect ratio regardless of the amount by which the diameter of tubular body 110 is increased.

Base member 100 may be expanded, for example, by use of a balloon catheter, in a procedure similar to the placement of intracoronary stents in the field of interventional cardiology. As tubular body 110 is dilated with a balloon (or other device), the distance between mounting elements 180 increases from distance D (FIG. 6) to a new distance D' (FIG. 7). The increased distance between mounting elements 180 allows a valve member 20 of larger dimensions to be coupled to base member 100. In this regard, the diameter of tubular body 110 is increased to accept a valve frame 30 having a larger diameter.

Securing element 160 (FIGS. 10 and 11) operates in substantially the same manner as securing element 140 described above in order to substantially maintain the aspect ratio. However, securing element 170 (FIGS. 12 and 13) does not substantially maintain the aspect ratio as the diameter of tubular body 110 is increased, since band 172 is directly attached to body 110 as described above. Accordingly, for the embodiment of base member 100 having securing elements 170, fingers 50 of valve frame 30 may require an increased length in order to be captured by band 172 when base member 100 is unexpanded (i.e., tubular body 110 has a small diameter).

Operation of cardiovascular assembly 10 will now be described in detail with reference to the embodiment shown in FIGS. 2 and 3. A base member 100 is installed into the body of a patient (e.g., a pulmonary artery) by conventionally known means. It is contemplated that base member 100 is delivered percutaneously and deployed in place by way of catheter-based tools. Base member 100 may also be delivered by way of minimally invasive surgery. After installation of base member 100, tubular body 110 may dilated to a small diameter during a first procedure. A valve member 20 having a small diameter valve frame 30 can be docked with base member 100 by insertion of fingers 50 into opening 154 defined by strap 152 and the engagement of coupling element 80 with mounting element 180, as described in detail above.

It is anticipated that valve member 20 with the small diameter valve frame 30 will be useable by a child through the first few years of life. After the child outgrows the small diameter valve frame 30, valve member 20 is detached from base member 100 by disengaging coupling element 80 from mounting element 180 and removing fingers 50 from the opening 154 defined by strap 152. FIG. 2 illustrates the removal of a valve member 20 having a small diameter valve frame 30. Following removal of valve member 20 from base member 100, tubular body 110 of base member 100 may be further dilated (e.g., with use of a balloon) to increase the diameter thereof. The diameter of body 110 can be determined with good precision by use of the dilating balloon. A valve member 20 having a larger diameter valve frame 30 may then be installed in a manner similar to the installation of the original valve member 20 having the small diameter valve frame 40. FIG. 3 illustrates the installation of a valve member 20 having a large diameter valve frame 30 after dilation of tubular body 110 to increase the diameter thereof. It should be appreciated that the diameter of tubular body 110 can be sequentially increased to receive a series of valve members 20 having incrementally larger diameter valve frames 30.

Valve frame 30 can be made to incremental diameter sizes, thereby allowing valve member 20 to be readily fit to a base member 100 having a tubular body 110 of an increased diameter. Valve member 20 can be repeatedly "upsized" as a child grows by repeatedly enlarging or dilating tubular body 110, and replacing the current valve member 20 with a new valve member 20 having a larger diameter valve frame 30. Thus, cardiovascular valve assembly 10 of the present invention can take a child through the early stages of life onto adolescence and adulthood.

The foregoing description is a specific embodiment of the present invention. It should be appreciated that this embodiment is described for purposes of illustration only, and that numerous alterations and modifications may be practiced by those skilled in the art without departing from the scope of the invention. It is intended that all such modifications and alterations be included insofar as they come within the scope of the invention as claimed or the equivalents thereof.

## Claims

1. A valve assembly comprising:
a base member (100) including:
an expandable tubular body (110) that is deformable to enlarge a diameter thereof,
a plurality of mounting elements (180) attached to the tubular body (110), and
a valve member (20) detachably mountable to the base member (100), wherein the valve member (20) includes:
a valve frame (30),
a plurality of leaflets (22) supported by the valve frame (30), and
a plurality of coupling elements (80) attached to the valve frame (30), each said coupling element (80) engageable with a respective mounting element (180) of said base member (100) to detachably mount said valve member (20) to said base member (100),
**characterized by**
a plurality of securing elements (140; 160; 170) attached to the tubular body, said securing elements (140; 160; 170) movable relative to the tubular body (110) to substantially maintain an aspect ratio D/H as the diameter of the tubular body (110) is enlarged, wherein D is the distance between adjacent mounting elements (180) and H is the distance between the mounting elements (180) and the securing elements (140; 160; 170),
wherein the plurality of securing elements (140; 160; 170) of the base member (100) maintain the valve member (20) in engagement with the base member (100).

2. A valve assembly according to claim 1, wherein said tubular body (110) is a mesh comprised of a plurality of intersecting elongate members (122, 124), said elongate members (122, 124) intersecting at intersection points (126).

3. A valve assembly according to claim 1, wherein said diameter of said tubular body (110) is sequentially enlarged to receive a series of valve members (20) having valve frames (30) of incrementally larger diameter.

4. A valve assembly according to claim 1, wherein said valve member (20) includes fingers (50) extending from the valve frame (30), said fingers (50) captured by said plurality of securing elements (140; 160; 170) when said valve member (20) is mounted to said base member (100).

5. A valve assembly according to claim 4, wherein each said securing element (140; 160; 170) includes:
a pair of arms (142) pivotally connected to said tubular body (110), said arms (142) moveable relative to each other when the diameter of said tubular body (110) is enlarged; and
a strap (152) extending between said pair of arms (142) to define an opening dimensioned to receive one of said fingers (50).

6. A valve assembly according to claim 4, wherein each said securing element (140; 160; 170) includes:
a pair of arms (142) pivotally connected to said tubular body (110), said arms (142) moveable relative to each other when the diameter of said tubular body (110) is adjusted; and
a band attached (162) to said pair of arms (142) to define an opening dimensioned to receive one of said fingers (50).

7. A valve assembly according to claim 4, wherein each said securing element (140; 160; 170) includes:
a band (162) attached to said tubular body (110) to define an opening dimensioned to receive one of said fingers (50).

## Patentansprüche

1. Ventilanordnung umfassend:
eine Basiseinheit (100) mit:
einem erweiterbaren schlauchförmigen Körper (110), welcher verformbar ist, um seinen Durchmesser zu vergrößern,
einer Vielzahl von Befestigungselementen (180), welche an dem schlauchförmigen Körper (110) befestigt sind, und
einer Ventileinheit (20), die abnehmbar an der Basiseinheit (100) befestigbar ist, wobei die Ventileinheit (20) beinhaltet:
einen Ventilrahmen (30),
eine Vielzahl von Blättern (22), welche durch den Ventilrahmen (30) gehalten werden, und
eine Vielzahl von Verbindungselementen (80), welche an dem Ventilrahmen (30) befestigt sind, wobei jedes Verbindungselement (80), das mit einem entsprechenden Befestigungselement (180) der Basiseinheit (100) verbindbar ist, ausgebildet ist, um die Ventileinheit (20) an der Basiseinheit (100) lösbar zu befestigen,
**gekennzeichnet durch**,
eine Vielzahl von Sicherungselementen (140; 160; 170), welche mit dem schlauchförmigen Körper verbunden sind, wobei die Sicherungselemente (140; 160; 170) relativ zu dem schlauchförmigen Körper (110) bewegbar sind, um das Verhältnis D/H im Wesentlichen beizubehalten, während der Durchmesser des schlauchförmigen Körpers (110) vergrößert wird, wobei D der Abstand zwischen angrenzenden Befestigungselementen (180) ist und H der Abstand zwischen den Befestigungselementen (180) und den Sicherungselementen (140; 160; 170) ist,
wobei die Vielzahl von Sicherungselementen (140; 160; 170) der Basiseinheit (100) die Verbindung der Ventileinheit (20) mit der Basiseinheit (100) beibehält.

2. Ventilanordnung gemäß Anspruch 1, wobei der schlauchförmige Körper (110) ein Netz ist, welches eine Vielzahl von sich kreuzenden länglichen Elementen (122, 124) umfasst, wobei die länglichen Elemente (122, 124) sich bei Kreuzungspunkten (126) kreuzen.

3. Ventilanordnung gemäß Anspruch 1, wobei der Durchmesser des schlauchförmigen Körpers (110) aufeinanderfolgend vergrößert wird, um eine Reihe von Ventileinheiten (20) aufzunehmen, welche Ventilrahmen (30) mit inkrementell größerem Durchmesser haben.

4. Ventilanordnung gemäß Anspruch 1, wobei die Ventileinheit (20) Finger (50) umfasst, welche sich von dem Ventilrahmen (30) aus erstrecken, wobei die Finger (50) durch die Vielzahl der Sicherungselemente (140; 160; 170) gehalten sind, wenn die Ventileinheit (20) an der Basiseinheit (100) befestigt ist.

5. Ventilanordnung gemäß Anspruch 4, wobei jedes Sicherungselement (140; 160; 170) umfasst:
ein Paar Arme (142), die gelenkig mit dem schlauchförmigen Körper (110) verbunden sind, wobei die Arme (142) relativ zueinander bewegbar sind, wenn der Durchmesser des schlauchförmigen Körpers (110) vergrößert wird; und
ein Gurt (152), welcher sich zwischen dem Paar Armen (142) erstreckt, um eine Öffnung zu definieren, die so dimensioniert ist, um einen der Finger (50) aufzunehmen.

6. Ventilanordnung gemäß Anspruch 4, wobei jedes Sicherungselement (140; 160; 170) umfasst:
ein Paar Arme (142), die gelenkig mit dem schlauchförmigen Körper (110) verbunden sind, wobei die Arme (142) relativ zueinander bewegbar sind, wenn der Durchmesser des schlauchförmigen Körpers (110) angepasst wird; und
ein Band (162), das an dem Paar Armen (142) befestigt ist, um eine Öffnung zu definieren, die so dimensioniert ist, um einen der Finger (50) aufzunehmen.

7. Ventilanordnung gemäß Anspruch 4, wobei jedes Sicherungselement (140; 160; 170) umfasst:
ein Band (162), das an dem schlauchförmigen Körper (110) befestigt ist, um eine Öffnung zu definieren, die so dimensioniert ist, um einen der Finger (50) aufzunehmen.

## Revendications

1. Assemblage de valve comprenant :
un élément de base (100) comprenant :
un corps tubulaire (110) expansible qui peut être déformé pour
agrandir un diamètre de celui-ci,
une pluralité d'éléments de montage (180) fixés au corps tubulaire (110), et
un élément de valve (20) pouvant être monté de manière détachable sur l'élément de base (100), dans lequel l'élément de valve (20) comprend :
un cadre de valve (30),
une pluralité de lames (22) supportées par le cadre de valve (30),
et
une pluralité d'éléments d'accouplement (80) fixés au cadre de valve (30), chacun desdits éléments d'accouplement (80) pouvant être rattaché à un élément de montage (180) respectif dudit élément de base (100) pour monter de manière détachable ledit élément de valve (20) sur ledit élément de base (100),
**caractérisé par**
une pluralité d'éléments de fixation (140 ; 160 ; 170) fixés au corps tubulaire, lesdits éléments de fixation (140 ; 160 ; 170) étant mobiles par rapport au corps tubulaire (110) pour maintenir sensiblement un rapport d'aspect D/H alors que le diamètre du corps tubulaire (110) est augmenté, où D est la distance entre des éléments de montage (180) adjacents et H est la distance entre les éléments de montage (180) et les éléments de fixation (140 ; 160 ; 170),
dans lequel la pluralité d'éléments de fixation (140 ; 160 ; 170) de l'élément de base (100) maintient l'élément de valve (20) en prise avec l'élément de base (100).

2. Assemblage de valve selon la revendication 1, dans lequel ledit corps tubulaire (110) est un filet composé d'une pluralité d'éléments allongés (122, 124) se croisant, lesdits éléments allongés (122, 124) se croisant en des points d'intersection (126).

3. Assemblage de valve selon la revendication 1, dans lequel ledit diamètre dudit corps tubulaire (110) est agrandi séquentiellement pour recevoir une série d'éléments de valve (20) ayant des cadres de valve (30) de diamètre incrémentalement plus grand.

4. Assemblage de valve selon la revendication 1, dans lequel ledit élément de valve (20) comprend des doigts (50) s'étendant à partir du cadre de valve (30), lesdits doigts (50) étant capturés par ladite pluralité d'éléments de fixation (140 ; 160 ; 170) lorsque ledit élément de valve (20) est monté sur ledit élément de base (100).

5. Assemblage de valve selon la revendication 4, dans lequel chacun desdits éléments de fixation (140 ; 160 ; 170) comprend :
une paire de bras (142) reliés de manière pivotante audit corps tubulaire (110), lesdits bras (142) étant mobiles l'un par rapport à l'autre lorsque le diamètre dudit corps tubulaire (110) est augmenté ; et
une sangle (152) s'étendant entre lesdits deux bras (142) pour définir une ouverture dimensionnée pour recevoir l'un desdits doigts (50).

6. Assemblage de valve selon la revendication 4, dans lequel chacun desdits éléments de fixation (140 ; 160 ; 170) comprend :
une paire de bras (142) reliés de manière pivotante audit corps tubulaire (110), lesdits bras (142) étant mobiles l'un par rapport à l'autre lorsque le diamètre dudit corps tubulaire (110) est ajusté ; et
une bande (162) fixée à ladite paire de bras (142) pour définir une ouverture dimensionnée pour recevoir l'un desdits doigts (50).

7. Assemblage de valve selon la revendication 4, dans lequel chacun desdits éléments de fixation (140 ; 160 ; 170) comprend :
une bande (162) fixée audit corps tubulaire (110) pour définir une ouverture dimensionnée pour recevoir l'un desdits doigts (50).
